# FASCICULE DE BREVET EUROPEEN

(11) **EP 3 569 258 B1**
(45) Date de publication et mention de la délivrance du brevet: **18.01.2023**
(21) Numéro de dépôt: 18172151.5
(22) Date de dépôt: 14.05.2018
(51) Int. Cl.: A61L 2/28

(54) **PROCEDE DE VALIDATION DU CYCLE DE TRAITEMENT D'UN PORTE INSTRUMENT DYNAMIQUE**
VALIDIERUNGSVERFAHREN EINES BEARBEITUNGSZYKLUS EINES DYNAMISCHEN WERKZEUGTRÄGERS
METHOD FOR VALIDATING THE TREATMENT CYCLE OF A DYNAMIC INSTRUMENT HOLDER

(43) Date de publication de la demande: 20.11.2019
(73) Titulaire: Slupecki, Patrice, 45000 Orléans (FR); Slupecki, Annie, 45000 Orléans (FR)
(72) Inventeur: SLUPECKI, Patrice, 45000 ORLEANS (FR); SLUPECKI, Annie, 45000 ORLEANS (FR); BRISSET, Lucien, 67580 MIETESHEIM (FR)
(74) Mandataire: Lequien, Philippe

(56) Documents cités:
- EP-A1- 0 631 610
- EP-B1- 0 631 610
- JP-A- S 534 604
- JP-A- 2009 075 084
- Damien Offner ET AL: "Cleaning of Dental Handpieces: A Method to Test its Efficiency, and its Evaluation With a Washer-DisinfectorLubricator-Dryer", Dentistry - Open Journal, vol. 3, no. 1, 29 November 2016 (2016-11-29), pages 10-16, XP055761137, DOI: 10.17140/DOJ-3-129

## Description

La présente invention entre dans le domaine du traitement des instruments chirurgicaux de type « Porte - instruments dynamiques » dits PID.

On entend par le terme « PID » l'ensemble des dispositifs médicaux d'utilisation quotidienne en cabinet dentaire, tel que par exemple turbines, contre-angles, pièce à main de chirurgie, à ultrasons ou les contre-angles de prophylaxie.

Ces PID sont des dispositifs qui génèrent un mouvement, par exemple de rotation ou d'oscillation, d'un instrument, notamment de type fraise, polissoir ou insert.

Lors de leur utilisation par un opérateur, les parties interne et externe des PID en contact avec le patient sont souillées, il convient de les nettoyer et de les désinfecter soigneusement.

Il est à noter que les PID présentent généralement une structure complexe. Ils comprennent notamment au sein de leur partie interne, plusieurs pièces constituées de différents matériaux, tels que par exemple des engrenages, des roulements à billes, des griffes de serrage, des boutons poussoirs, des conduits ou buses d'injections ou encore des coudes.

De manière contraignante, l'ensemble des PID connus n'étant pas à parois étanches, lors de leur utilisation, les protéines de sang, la salive, des fragments osseux et de chair peuvent entrer dans la partie interne des PID et contaminer lesdites pièces.

En outre, le nombre, la nature des matériaux, les caractéristiques physico-chimiques, l'accessibilité et la diversité des pièces, présentes en partie interne des PID, rendent leur nettoyage difficile.

A cela s'ajoute une contrainte supplémentaire à savoir que les PID ne peuvent être ni immergés dans une solution nettoyante ni traités dans un bac à ultrasons.

Ainsi, pour contrôler le nettoyage des parties internes des PID, il est nécessaire de les démonter, la structure des PID étant complexe, le démontage est long et fastidieux.

C'est pourquoi, il existe des protocoles spécifiques pour nettoyer les PID.

De manière usuelle, avant de réutiliser un PID, il convient de le traiter, c'est-à-dire de le laver, désinfecter et de le stériliser par un protocole d'hygiène spécifique.

Généralement, le protocole de traitement des PID pour le lavage et la désinfection consiste à réaliser les étapes suivantes, suivant la norme NF-EN-ISO 15-883-1 et NF-EN-ISO 15-883-2 :
- A) Pré désinfecter la surface externe du PID immédiatement après son utilisation, par exemple avec une lingette désinfectante
- B) Rincer la partie interne et externe du PID, par exemple en aspergeant et rinçant l'intérieur et l'extérieur des PID avec une solution spécifique à une température inférieure à 45°C,
- C) Nettoyer la partie interne et externe du PID, avec mise en rotation interne, de la même manière que précédemment en appliquant une solution nettoyante à une température donnée et un temps donné
- D) Désinfecter la partie interne et externe du PID, de manière chimique ou thermique
- E) Sécher la partie interne et externe du PID,
- F)) Lubrifier le PID par exemple en propulsant une goutte d'huile par de l'air sous pression
- G) Stériliser le PID, par exemple dans un autoclave.

Une partie de ces étapes peut être réalisée par des automates spécifiques capables de réaliser successivement et dans l'ordre une partie ou l'intégralité des étapes B) à G) susmentionnés.

On connait par exemple un dispositif automatique de désinfection de moteurs chirurgicaux, qui permet de réaliser les étapes B) à F) susmentionnées.

Néanmoins, ce type d'automate ne permet pas d'analyser et de contrôler la réalisation effective et conforme de chacune de ces étapes. A ce jour, la seule possibilité de vérifier que le PID a été correctement nettoyé et/ou désinfecté dans l'automate, en particulier dans sa partie interne, est de le démonter manuellement après son passage dans l'automate.

De la même manière, il n'existe pas de moyen pour contrôler la qualité de séchage et la lubrification adéquate du PID suite à son passage au sein de l'automate.

Le démontage et le remontage manuel d'un PID étant complexe et long, il constitue une réelle perte de temps, sachant que le besoin de nettoyage et de désinfection peut concerner quelques dizaines de PID par jour à plusieurs centaines en fonction du site concerné.

C'est pourquoi, il convient de trouver une solution alternative au démontage qui soit rapide, efficace, et qui permet de contrôler la qualité de lavage, nettoyage, et désinfection interne et externe d'un PID en sortie d'automate.

La présente invention a pour but de pallier les inconvénients de l'état de la technique, en proposant un procédé de validation du cycle de traitement, selon l'objet de la revendication 1

Ainsi, la réalisation du procédé de l'invention va permettre à un opérateur, en visualisant simplement le PID test, de constater, par simple observation du matériau transparent du PID, que les opérations de lavage et de désinfection ont bien eu lieu au sein de l'automate. Ainsi, par simple visualisation de la paroi du PID test redevenu intégralement transparente à l'œil nu, l'opérateur va pouvoir valider le cycle de traitement et sa réalisation au sein de l'automate. Grâce au procédé de l'invention, on va pouvoir constater que le PID a été lavé et désinfecté au niveau de sa paroi aussi bien sur la surface interne que sur sa surface externe, ceci sans nécessiter son démontage.

Ainsi, si on réalise le procédé de l'invention au sein d'un automate qui comprend, en plus du PID test, des PID à laver, on va, par analogie, pouvoir valider le bon nettoyage, lavage et désinfection des PID à laver. Il ne sera plus alors nécessaire de démonter les PID à laver en sortie d'automate pour vérifier la conformité du nettoyage.

De cette manière, ce procédé permet de gagner du temps sur le contrôle de la qualité de nettoyage des PID en sortie d'automate.

De plus, selon d'autres caractéristiques de la revendication 1 :
- préalablement à l'étape a) du procédé de validation du cycle de traitement de l'invention, on enduit de souillure la surface interne et la surface externe dudit PID test,
- lesdites souillures sont de type protéique,
- ledit réactif est un réactif colorimétrique destiné à interagir avec les souillures pour donner une substance colorée modifiant les propriétés de transparence du matériau de la paroi du PID test souillé.

De cette manière, avant démarrage de l'étape b) où on intègre le PID test au sein de l'automate, le PID test souillé avec des protéines présente une paroi visuellement colorée, due à l'interaction entre les protéines et le réactif. Le PID test que l'on intègre dans l'automate ne présente plus une paroi visuellement transparente.

Selon d'autres caractéristiques de l'invention :
- après l'étape f) de validation de la réalisation du cycle de traitement, on réalise l'étape g) dans laquelle:
- on applique, sur la paroi interne et/ou externe transparente du PID test, un révélateur protéique modifiant les propriétés de transparence de la paroi en présence de souillures,
- on constate la disparition complète des souillures, de type protéique, de la paroi du PID test par une absence de modification des propriétés de transparence de la paroi du PID test en présence du révélateur,
- par exemple, ledit révélateur consiste en une solution au Bleu de Coomassie ou en de la ninhydrine.

L'étape g) permet de confirmer la disparition des souillures protéiques de la paroi du PID test, et ainsi de confirmer une transparence de la paroi du PID test identique à celle avant souillure.

En outre, selon d'autres caractéristiques du procédé de l'invention,
- le cycle de traitement comprend, en plus, après les opérations de lavage et de désinfection, des opérations de stérilisation,
- au sein du PID test et/ou de l'automate se trouve une sonde de contrôle de la température.

L'invention a également pour objet un porte instruments dynamique test selon l'objet de la revendication 7.

D'autres caractéristiques et avantages de l'invention ressortiront de la description détaillée qui va suivre des modes de réalisation non limitatifs de l'invention.

La présente invention concerne un procédé de validation du cycle de traitement, comprenant des opérations de lavage et de désinfection, d'instruments chirurgicaux de type porte instruments dynamiques dit PID, au sein d'un automate.

Selon l'invention, on débute le procédé de l'invention en réalisant l'étape a) dans laquelle on dépose sur la surface interne et la surface externe de la paroi préalablement souillée d'un PID test en matériau transparent, un réactif modifiant visuellement les propriétés de transparence dudit matériau dudit PID test.

Ledit PID test présente une paroi intégralement en matériau transparent qui permet d'observer visuellement la propreté de la surface interne et externe de sa paroi et d'observer son contenu.

Selon l'invention, ledit PID test est dans un matériau transparent résistant mécaniquement au cycle de traitement ayant lieu au sein de l'automate. En d'autres termes, ledit matériau transparent du PID test est conçu dans un matériau transparent résistant aux conditions physicochimiques de nettoyage et désinfection, voire même de stérilisation, au sein d'un automate.

Par exemple, ledit matériau transparent du PID test consiste en type plastique ou verre transparent etc...

Avantageusement, la configuration et la structure du PID test est similaire à celle d'un PID traditionnel. Plus précisément, au sein du PID test on a simulé par exemple des engrenages, des roulements à billes, des coudes, des tubulures etc... pour être le plus proche possible de la configuration complexe d'un PID.

Selon un mode de réalisation particulier du procédé de l'invention, préalablement à l'étape a), on enduit de souillure la surface interne et la surface externe dudit PID test. Préférentiellement, lesdites souillures sont de type protéique.

Les souillures consistent notamment en des éléments biologiques imprégnés sur le matériau transparent du PID test au niveau de sa surface interne et de sa surface externe.

Par exemple, les souillures sont des protéines ou salissures biologiques, en particulier de type salive, protéines de sang colorées, fragments osseux ou de chair ou encore en des protéines d'œufs.

Lesdites souillures peuvent également être des protéines avec une étiquette réagissant avec le réactif, des protéines fluorescentes sous UV etc...

Ainsi, selon une particularité de l'invention, en plus d'être dans un matériau transparent, le PID test est capable de fixer ou de s'imprégner desdites souillures.

Lesdites souillures ont la capacité d'être éliminées de la paroi durant les opérations de lavage et de désinfection, par exemple sous l'action de fluides de nettoyage.

Selon l'invention, lesdites souillures, après imprégnation sur la paroi du PID test, sont capables de réagir chimiquement avec ledit réactif modifiant visuellement les propriétés de transparence dudit matériau dudit PID test.

Par exemple, si les souillures sont des protéines incolores et le réactif du Bleu de Coomassie, alors la paroi du PID test sera colorée en Bleu.

Selon un autre mode de réalisation, si les souillures sont des protéines de sang, alors la paroi du PID test sera visuellement rouge.

Selon un autre mode de réalisation, si les souillures sont des protéines de fusion avec une étiquette fluorescente, par exemple de type GFP dit « Green Fluroscent Protein », alors le réactif est la lumière UV, et la paroi du PID test sera en vert fluorescent sous UV.

Selon un mode de réalisation particulier, ledit réactif est un réactif colorimétrique destiné à interagir avec les souillures pour donner une substance colorée modifiant les propriétés de transparence du matériau de la paroi du PID test souillé.

Par exemple, ledit réactif consiste en du Bleu de Coomassie ou de la Ninhydrine, ou de la lumière UV, ou une enzyme destinée à réagir avec l'étiquette d'une protéine de fusion afin de modifier les propriétés de transparence de la paroi du PID test.

En d'autres termes, le réactif est capable d'interagir avec les protéines de souillures choisies et présentes sur la surface interne et externe du PID test de manière à donner une substance colorée ou une substance qui va modifier les propriétés de transparence de la paroi du PID test.

De manière préférentielle, on choisira un réactif dont la coloration est visible à l'œil nu, de type Bleu de Coomassie ou Ninhydrine et capable d'interagir avec les protéines imprégnées sur la paroi du PID test.

De cette manière, le PID souillé présentera une paroi intégralement couleur bleue ou respectivement violet foncé facilement détectable à l'œil nu.

Selon le procédé de l'invention, après l'étape a), on réalise dans cet ordre les étapes suivantes :
b) On intègre dans l'automate ledit PID test, dont la transparence de la paroi a été modifié par la présence des souillures réagissant avec ledit réactif
c) On réalise l'intégralité du cycle de traitement au sein de l'automate.

Selon l'invention, le cycle de traitement au sein de l'automate comprend au moins des opérations de lavage et de désinfection. Ces opérations de lavage et de désinfection consistent à mettre en contact le PID avec des fluides et des solutions nettoyantes et désinfectantes aussi bien dans sa partie interne que dans sa partie externe.

Par exemple, les solutions nettoyantes consistent en des solutions détergentes pouvant être enzymatiques et également des tensioactifs.

Par exemple, les opérations de désinfection consistent à faire passer de l'eau au sein de l'automate soit par exemple 60 secondes à 90°C ou 180 secondes à 85°C.

Avantageusement, et pour contrôler les opérations de désinfection au sein de l'automate, c'est-à-dire pour valider le cycle de traitement du mode de réalisation particulier, une sonde de contrôle de la température peut être placée au sein du PID test et/ou dans l'enceinte de l'automate.

Cette sonde permet au cours du cycle de désinfection de contrôler la température atteinte dans la partie interne du PID test et/ou dans l'enceinte de l'automate.

Ainsi, cette sonde permet de valider ou non le bon déroulement de l'opération de lavage et/ou de désinfection du cycle de traitement.

Selon le procédé de l'invention, la bonne réalisation du cycle de traitement, plus spécifiquement les opérations de lavage et de désinfection de la partie interne et externe des PID, est validée par la mise en œuvre, consécutivement aux étapes a) à c), des étapes d) et f)) ci-dessous :
d) On observe, en fin de cycle de traitement, la paroi dudit PID test,
e) on compare les propriétés de transparence de la paroi du PID test avec celles avant souillure et ajout du réactif,
f) On valide la réalisation du cycle de traitement lorsque l'intégralité de la paroi dudit PID test présente des propriétés de transparence du matériau identiques à celles avant souillure et ajout du réactif.

Ainsi, la réalisation du procédé de l'invention permet par un simple contrôle visuel de vérifier que la surface interne et la surface externe de PID ont été lavées et désinfectées durant le cycle de traitement. En effet, le fait que la paroi du PID test retrouve un aspect identique à celui avant souillure et ajout du réactif est synonyme d'un PID propre.

Le PID test en sortie d'automate qui présente une paroi intégralement transparente à l'œil nu signifie une absence de souillure et leur élimination par les fluides de nettoyage et de désinfection. Par analogie, si le cycle de traitement a été validé pour le PID test, on peut également le valider pour les autres PID potentiellement présents dans l'automate.

La mise en œuvre du procédé de l'invention représente donc une solution rapide et efficace pour valider le cycle de traitement des PID au sein d'un automate sans avoir à démonter chacun d'entre eux.

En outre, pour confirmer ce que l'on constate à l'œil nu, c'est-à-dire pour confirmer le fait que la paroi du PID test a retrouvé un aspect de transparence identique à celui avant souillure, il est possible après l'étape f) de validation de la réalisation du cycle de traitement, de réaliser une étape g) supplémentaire.

Selon l'invention, cette étape g) consiste à :
- appliquer, sur la paroi interne et/ou externe transparente du PID test, un révélateur protéique modifiant les propriétés de transparence de la paroi en présence de souillures,
- constater la disparition et l'élimination complète des souillures, de type protéique, de la paroi du PID test par une absence de modification des propriétés de transparence de la paroi du PID test en présence du révélateur.

Selon un mode de réalisation particulier, l'application du révélateur se fait à l'aide d'un élément imprégné du révélateur, par exemple d'un coton tige ou d'une lingette, qu'on racle sur la paroi interne et externe du PID test.

Selon un mode de réalisation préférentiel, ledit révélateur consiste en une solution de ninhydrine. Dans ce cas si des salissures non détectées à l'œil nu sont encore présentes alors l'élément se colorera en violet foncé, dans le cas contraire une couleur vert clair persistera.

Selon un autre mode de réalisation, ledit révélateur consiste en du Bleu de Coomassie qui va se fixer sur la paroi à l'endroit où des salissures persistent encore.

Ainsi, ledit révélateur permet de confirmer la transparence sans souillure avant et après le cycle de traitement.

Selon un mode de réalisation particulier, le cycle de traitement de l'automate comprend, en plus et à la suite des opérations de lavage et de désinfection, des opérations de stérilisation.

## Revendications

1. Procédé de validation du cycle de traitement, comprenant des opérations de lavage et de désinfection, d'instruments chirurgicaux de type porte instruments dynamiques dit PID, au sein d'un automate,
**caractérisé en ce que** le procédé met en oeuvre un porte instruments dynamique test, dit PID test, comprenant une paroi intégralement en matériau transparent présentant une surface interne et une surface externe, le PID test étant destiné à présenter une configuration et une structure d'un PID traditionnel,
ladite paroi en matériau transparent du PID test étant destinée à permettre d'observer visuellement la propreté de la surface interne et de la surface externe de la paroi et d'observer le contenu du PID test,
et **en ce que** :
a) on dépose sur la surface interne et la surface externe de la paroi préalablement souillée du PID test, un réactif modifiant visuellement les propriétés de transparence dudit matériau dudit PID test,
b) on intègre dans l'automate ledit PID test,
c) on réalise l'intégralité du cycle de traitement au sein de l'automate,
d) on observe, en fin de cycle de traitement, la paroi dudit PID test,
e) on compare les propriétés de transparence de la paroi du PID test avec celles avant souillure et ajout du réactif,
f) on valide la réalisation du cycle de traitement lorsque l'intégralité de la paroi dudit PID test présente des propriétés de transparence du matériau identiques à celles avant souillure et ajout du réactif,
et **en ce que** si le cycle de traitement est validé pour le PID test, on valide la réalisation du cycle de traitement pour d'autres porte instruments dynamiques potentiellement présents dans l'automate,
et **en ce que** préalablement à l'étape a), on enduit de souillure la surface interne et la surface externe dudit PID test, lesdites souillures étant de type protéique,
et **en ce que** ledit réactif est un réactif colorimétrique destiné à interagir avec les souillures pour donner une substance colorée modifiant les propriétés de transparence du matériau de la paroi du PID test souillé.

2. Procédé de validation du cycle de traitement, selon la revendication 1, **caractérisé en ce que**, après l'étape f) de validation de la réalisation du cycle de traitement :
- on applique, sur la paroi interne et/ou externe transparente du PID test, un révélateur protéique modifiant les propriétés de transparence de la paroi en présence de souillures,
- on constate la disparition complète des souillures, de type protéique, de la paroi du PID test par une absence de modification des propriétés de transparence de la paroi du PID test en présence du révélateur.

3. Procédé de validation du cycle de traitement, selon la revendication précédente, **caractérisé en ce que** ledit révélateur consiste en une solution au Bleu de Coomassie ou en de la ninhydrine.

4. Procédé de validation du cycle de traitement, selon l'une quelconque des revendications précédentes, **caractérisé en ce que** le cycle de traitement comprend, en plus, après les opérations de lavage et de désinfection, des opérations de stérilisation.

5. Procédé de validation du cycle de traitement, selon la revendication précédente, **caractérisé en ce qu'**au sein du PID test et/ou de l'automate se trouve une sonde de contrôle de la température.

6. Procédé de validation du cycle de traitement, selon l'une quelconque des revendications précédentes, **caractérisé en ce que** le procédé de validation est réalisé au sein d'un automate qui comprend, en plus du PID test, des PID à laver.

7. Porte instruments dynamique test, dit PID test, pour un procédé de validation du cycle de traitement, comprenant des opérations de lavage et de désinfection, d'instruments chirurgicaux de type porte instruments dynamiques dit PID, le PID test présentant :
- une configuration et une structure d'un porte instruments dynamique traditionnel susceptible de devoir être démonté manuellement après un passage dans un automate pour vérifier qu'il a été correctement nettoyé et/ou désinfecté dans l'automate ;
- une paroi intégralement en matériau transparent permettant d'observer visuellement la propreté d'une surface interne et d'une surface externe de la paroi et d'observer le contenu du PID test.

## Patentansprüche

1. Verfahren zur Validierung des Bearbeitungszyklus, umfassend Reinigungs- und Desinfektionsmaßnahmen von chirurgischen Instrumenten vom Typ dynamischer Instrumententräger, bezeichnet als PID, innerhalb eines Automaten,
**dadurch gekennzeichnet, dass** das Verfahren einen dynamischen Test-Instrumententräger, bezeichnet als Test-PID, verwendet, der eine Wand umfasst, die vollständig aus transparentem Material ist, wobei sie eine innere Oberfläche und eine äußere Oberfläche aufweist, wobei der Test-PID bestimmt ist, eine Konfiguration und eine Struktur eines herkömmlichen PID aufzuweisen,
wobei die Wand aus transparentem Material des Test-PID bestimmt ist, die Sauberkeit der inneren Oberfläche und der äußeren Oberfläche der Wand visuell zu beobachten und den Inhalt des Test-PID zu beobachten,
und dass:
a) auf der inneren Oberfläche und der äußeren Oberfläche der zuvor verschmutzten Wand des Test-PID ein Reagens aufgebracht wird, das die Transparenzeigenschaften des Materials des Test-PID visuell verändert,
b) der Test-PID in den Automaten integriert wird,
c) der gesamte Bearbeitungszyklus innerhalb des Automaten durchgeführt wird,
d) am Ende des Bearbeitungszyklus die Wand des Test-PID beobachtet wird,
e) die Transparenzeigenschaften der Wand des Test-PID mit denen vor der Verschmutzung und Hinzufügung des Reagens verglichen werden,
f) die Durchführung des Bearbeitungszyklus validiert wird, wenn die gesamte Wand des Test-PID Transparenzeigenschaften des Materials aufweist, die mit denen vor der Verschmutzung und Hinzufügung des Reagens identisch sind,
und dass, wenn der Bearbeitungszyklus für den Test-PID validiert ist, die Durchführung des Bearbeitungszyklus für andere dynamische Instrumententräger validiert wird, die potentiell in dem Automaten vorhanden sind,
und dass vor dem Schritt a) die innere Oberfläche und die äußere Oberfläche des Test-PID verschmutzt werden, wobei die Verschmutzungen vom Typ Protein sind,
und dass das Reagens ein kolorimetrisches Reagens ist, das bestimmt ist, mit den Verschmutzungen zu interagieren, um eine kolorierte Substanz abzugeben, die die Transparenzeigenschaften des Materials der Wand des verschmutzten Test-PID verändert.

2. Validierungsverfahren des Bearbeitungszyklus nach Anspruch 1, **dadurch gekennzeichnet, dass** nach dem Schritt f) der Validierung der Durchführung des Bearbeitungszyklus:
- auf die innere und/oder äußere transparente Wand des Test-PID ein Proteinnachweis aufgetragen wird, der die Transparenzeigenschaften der Wand bei Anwesenheit von Verschmutzungen verändert,
- das vollständige Verschwinden der Verschmutzungen vom Proteintyp von der Wand des Test-PID durch eine Abwesenheit von Veränderung der Transparenzeigenschaften der Wand des Test-PID bei Anwesenheit des Nachweises festgestellt wird.

3. Validierungsverfahren des Bearbeitungszyklus nach vorangehendem Anspruch, **dadurch gekennzeichnet, dass** der Nachweis aus einer Coomassie-Brillantblau-Lösung oder aus Ninhydrin besteht.

4. Validierungsverfahren des Bearbeitungszyklus nach einem der vorangehenden Ansprüche, **dadurch gekennzeichnet, dass** der Bearbeitungszyklus zusätzlich nach den Reinigungs- und Desinfektionsmaßnahmen Sterilisationsmaßnahmen umfasst.

5. Validierungsverfahren des Bearbeitungsverfahrens nach vorangehendem Anspruch, **dadurch gekennzeichnet, dass** sich innerhalb des Test-PID und/oder des Automaten eine Sonde zur Überwachung der Temperatur befindet.

6. Validierungsverfahren des Bearbeitungsverfahrens nach einem der vorangehenden Ansprüche, **dadurch gekennzeichnet, dass** das Validierungsverfahren innerhalb eines Automaten durchgeführt wird, der neben dem Test-PID zu reinigende PID umfasst.

7. Dynamischer Test-Instrumententräger, bezeichnet als Test-PID, für ein Validierungsverfahren des Bearbeitungszyklus, der Reinigungs- und Desinfektionsmaßnahmen von chirurgischen Instrumenten vom Typ dynamischer Instrumententräger, bezeichnet als PID, umfasst, wobei der Test-PID aufweist:
- eine Konfiguration und eine Struktur eines herkömmlichen dynamischen Instrumententrägers, der nach einer Behandlung in einem Automaten manuell demontiert werden muss, um zu überprüfen, dass er in dem Automaten korrekt gereinigt und/oder desinfiziert wurde;
- eine Wand, die vollständig aus transparentem Material ist, die erlaubt, die Sauberkeit einer inneren Oberfläche und einer äußeren Oberfläche der Wand visuell zu beobachten und den Inhalt des Test-PID zu beobachten.

## Claims

1. A treatment cycle validation method, comprising operations of washing and disinfecting surgical instruments of the dynamic instrument-holder, DIH, type within an automaton,
**characterised in that** the method implements a test dynamic instrument-holder, test DIH, comprising a wall fully made of transparent material having an inner surface and an outer surface, the test DIH being intended to have a configuration and a structure of a traditional DIH,
said wall made of transparent material of the test DIH being intended to enable visual observation of the cleanliness of the inner surface and the outer surface of the wall and observation of the content of the test DIH, and by:
a) depositing onto the inner surface and the outer surface of the previously soiled wall of the test DIH, a reagent visually modifying transparency properties of said material of said test DIH,
b) integrating said test DIH into the automaton,
c) executing the entire treatment cycle within the automaton,
d) observing, at the end of the treatment cycle, the wall of said test DIH,
e) comparing transparency properties of the wall of the test DIH with those before soiling and adding the reagent,
f) validating the execution of the treatment cycle when the entire wall of said test DIH has transparency properties of the material identical to those before soiling and adding the reagent,
and by, if the treatment cycle is validated for the test DIH, validating the execution of the treatment cycle for other dynamic instrument-holders potentially present in the automaton,
and by, prior to step a), coating the inner surface and the outer surface of said test DIH with soil, said soil being of the protein type,
and **in that** said reagent is a colorimetric reagent intended to interact with the soil to yield a coloured substance modifying transparency properties of the material of the wall of the test DIH soiled.

2. The treatment cycle validation method according to claim 1, **characterised by**, after step f) of validating the execution of the treatment cycle:
- applying, to the inner and/or outer transparent wall of the test DIH, a protein developer modifying transparency properties of the wall in the presence of soil,
- noticing total disappearance of the protein type soil, from the wall of the test DIH by an absence of modification of the transparency properties of the wall of the test DIH in the presence of the developer.

3. The treatment cycle validation method according to the preceding claim, **characterised in that** said developer consists of a Coomassie Blue solution or of ninhydrin.

4. The treatment cycle validation method according to any of the preceding claims, **characterised in that** the treatment cycle additionally comprises, after the washing and disinfection operations, sterilisation operations.

5. The treatment cycle validation method according to the preceding claim, **characterised in that** within the test DIH and/or the automaton there is a temperature control probe.

6. The treatment cycle validation method according to any of the preceding claims, **characterised in that** the validation method is executed within an automaton which comprises, in addition to the test DIH, DIHs to be washed.

7. A test dynamic instrument-holder, test DIH, for a treatment cycle validation method, comprising operations of washing and disinfecting surgical instruments of the dynamic instrument-holder, DIH, type, the test DIH having:
- a configuration and a structure of a traditional dynamic instrument-holder likely to have to be manually disassembled after passing through an automaton to check that it has been properly cleaned and/or disinfected in the automaton;
- a wall fully made of transparent material enabling visual observation of the cleanliness of an inner surface and an outer surface of the wall and observation of the content of the test DIH.
